# EUROPEAN PATENT APPLICATION

(11) **EP 1 344 515 A1**
(43) Date of publication of application: **17.09.2003**
(21) Application number: 01272267.4
(22) Date of filing: 20.12.2001
(51) Int. Cl.: A61K 7/00, A61K 7/40, A61K 7/42

(54) **SKIN COSMETICS**

(30) Priority: 22.12.2000 JP 2000390953
(71) Applicant: Shu Uemura Cosmetics Inc., Tokyo 107-0062 (JP); Kaise, Chihiro, Tokyo 156-0054 (JP); Kobayashi, Masako, Tokyo 156-0054 (JP); Kaneko, Teruhisa, Tokyo 156-0054 (JP); Honda, Shinkichi, Nagareyama-shi, Chiba 270-0115 (JP)
(72) Inventor: KAISE, Chihiro, c/o SHU UEMURA COSMETICS INC. LAB., Tokyo 156-0054 (JP); KOBAYASHI, M., c/o SHU UEMURA COSMETICS INC. LAB., Tokyo 156-0054 (JP); KANEKO, T., c/o SHU UEMURA COSMETICS INC. LAB., Tokyo 156-0054 (JP); HONDA, Shinkichi, Nagareyama-shi, Chiba 270-0115 (JP)
(74) Representative: de Roquemaurel, Bruno
(86) International application number: JP0111182
(87) International publication number: WO02051361

(57) **Abstract**

Skin care products for alleviating problems caused by deterioration of skin immunity. Provided are:
(1) A skin care product containing as essential ingredients an N-acetylated basic amino acid compound and an antioxidant; and
(2) A skin care product containing as essential ingredients at least one selected from an N-acetylated basic amino acid compound, an antioxidant, and deep sea water, and yeast and/or yeast extracts.

## Description

### TECHNICAL FIELD

The present invention relates to a skin care product for preventing deterioration of skin immunity caused by factors such as aging.

### BACKGROUND ART

Numerous factors are known to cause deterioration of skin immunity, including excessive mental stress, exposure to high dosages of UV-light, and aging. As the skin's immunity deteriorates, many health problems arise. For example, stratum corneum loses its barrier function, making skin permeable to allergens. Also, the immunity mediated by Langerhans' islets deteriorates when skin is exposed to UV-radiation. This leads to progression of atopic dermatitis. Furthermore, as skin ages, response to dinitrochlorobenzene, a typical contact allergen, is reduced, as is the number of dendritic epidermal cells, which are known to act as natural killer cells. Among the skin-related problems are occurrence of dry skin, slowing of skin turnover, and emergence of wrinkles.

Some of the traditional skin care products intended for alleviating the problems caused by the deterioration of skin immunity contained moisturizers and UV-blocking agents, while others contained retinol-based material for the purpose of preventing aging. Conventional anti-aging skin care products could hardly do more than just cope with seasonal dry skin or skin troubles caused by a temporal decrease in skin activity.

Thus, no skin care product has ever been proposed that can effectively alleviate various symptoms resulting from deterioration of the skin immunity.

Accordingly, it is an objective of the present invention to provide a skin care product for alleviating the above-identified problems caused by the deterioration of the skin immunity.

### DISCLOSURE OF THE INVENTION

In the course of the present inventors' studies, they have made a finding that the problems associated with deterioration of skin immunity can be alleviated by using an N-acetylated basic amino acid compound or a β-glucan in combination with certain other components. This finding ultimately led the present inventors to complete the present invention.

Hence, the present invention concerns:
1. A skin care product containing as essential ingredients an N-acetylated basic amino acid compound and an antioxidant.
2. A skin care product containing as essential ingredients at least one selected from an N-acetylated basic amino acid compound, an antioxidant, and deep sea water, and yeast and/or yeast extracts.
3. The skin care product according to the invention as described in 1 or 2 above, wherein the N-acetylated basic amino acid compound includes at least one selected from the group consisting of N-acetylarginine, N-α-acetyllysine, N-ε-acetyllysine, N-α-acetylornithine, N-γ-acetylornithine, N-α-acetylhistidine, and salts thereof.
4. The skin care product according to the invention as described in 1 or 2 above, wherein the antioxidant includes at least one selected from the group consisting of red wine yeast extracts, white wine yeast extracts, hypotaurine, tocopherol, vanillin, catechin extracts, dibutylhydroxytoluene, coffee extracts, and dl-alpha-tocopherol acetate.

As used herein, the term "yeast and/or yeast extracts" is meant to encompass live yeast, powdered and dried yeast, and yeast extracts prepared by subjecting yeast to a solubilization, extraction, or purification process under proper conditions designed for autolysis, hot-water extraction, oxidative hydrolysis, and enzymatic digestion.

The yeast for use in the present invention are ones that include a β-glucan such as β-1,3-glucan and β-1,3/1,6-D-glucan with a molecular weight of 150,000 to 400,000, preferably 200,000 to 300,000. Examples of such yeast include those belonging to the *genus Saccharomyces,* such as brewer's yeast and baker's yeast.

The yeast extracts for use in the present invention may be obtained by physically, chemically, or biochemically processing the cells and may be selected independently of the type or origin of the yeast, or preparation methods used for extracting, purifying or treating the yeast. Commercially available dry yeast powders and yeast extracts are particularly convenient because of their availability and readiness in handling.

The above-described yeast or yeast extracts contain proteins, lipids, inorganic salts, organic acids, vitamins, amino acids, sugars, nucleic acid-related substances and other useful substances.

β-glucan, a principal component of the yeast and/or the yeast extract, has a polysaccharide backbone and thus acts as an excellent moisturizer. It also facilitates synthesis of collagen and thus physiological activity of skin. β-glucan further provides excellent anti-photoaging effects since its polysaccharide backbone serves to protect cells from UV-light and other harmful radiation that would otherwise kill the cells.

The amount of the yeast and/or the yeast extracts to be blended is from 0.0001 to 20wt%, and preferably from 0.005 to 0.1wt% with respect to the total amount of the entire components of the skin care product. Desired effects are not obtained if the amount is less than 0.0001wt% or larger than 20wt%.

The N-acetylated basic amino acid compound for use in the present invention may be a mono-acetylated basic amino acid obtained by acetylating amino group of a basic amino acid, or a salt thereof.

The basic amino acid that constitutes the N-acetylated basic amino acid compound may be arginine, lysine, ornithine, or histidine and may be L-form, D-form or racemic (DL) mixture of its optical isomers. These basic amino acids may be obtained through fermentation, synthesis, or any other production method.

Specific examples of the N-acetylated basic amino acid compound include N-acetylarginine, N-α-acetyllysine, N-ε-acetyllysine, N-α-acetylornithine, N-γ-acetylornithine, N-α-acetylhistidine, and salts thereof.

The N-acetylated basic amino acid compound for use in the present invention may be used in a free form, in which the carboxyl acid moiety and the basic moiety in an N-acetylated basic amino acid molecule are both free, or in the form of a salt of N-acetylated basic amino acid, or in the form of a mixture of the two forms.

Examples of basic salts include alkali metal salts such as those formed with sodium, potassium and lithium, alkali earth metal salts such as those formed with calcium and magnesium, ammonium salts, salts formed with amines such as monoethanolamine, triethanolamine and triisopropanolamine, salts of basic amino acids such as arginine, lysine, ornithine and histidine, salts formed with amino acid derivatives such as amino acid esters and acylated peptides, salts formed with phospholipids such as phosphatidylcholine (PC), phosphatidylethanolamine (PE) and phosphatidylglycerol (PG), salts formed with cationic surfactants such as cation polymers and alkyltrimethylammonium, and salts formed with amphoteric surfactants such as betaine. These basic components are used independently or as a mixture.

Examples of acidic salts include salts of inorganic acids such as hydrochloric acid, hydrobromic acid, sulfuric acid, nitric acid and phosphoric acid, salts of organic acids such as acetic acid, citric acid, p-toluenesulfonic acid, fatty acids, succinic acid, maleic acid, and trifluoroacetic acid, salts of acidic amino acids such as glutamic acid, aspartic acid and pyroglutamic acid, salts formed with amino acid derivatives such as acyl glutamic acid, acyl glutamine, acetyl glutamine, and acylated peptides, salts formed with phospholipids such as phosphatidylcholine (PC), phosphatidylethanolamine (PE) and phosphatidylglycerol (PG), salts formed with anionic surfactants such as lauryl sulfuric acid and polyoxylauryl sulfuric acid, and salts formed with amphoteric surfactants such as betaine. These acidic components are used independently or as a mixture.

By blending the N-acetylated basic amino acid compound into the skin care product, percutaneous absorption of the skin care product is enhanced. Also, basic amino acids that result from intradermal deacetylation of the N-acetylated amino acid compound are thought to facilitate syntheses of collagen and hyaluronic acid.

The amount of the N-acetylated basic amino acid compound to be blended is from 0.001 to 5.0%, and preferably from 0.01 to 3% with respect to the total amount of all of the components of the skin care product. Desired effects are not obtained if the amount is less than 0.001%, whereas the stability of the skin care product is reduced if the amount exceeds 5.0%.

The term "antioxidant" as used herein refers to a group of substances that can interrupt a reaction cascade in which free radicals produced during the oxidation chain reaction brought about by atmospheric oxygen react with oils and fats (lipids) present in sebum to form inactive materials. These substances act as a hydroxyl radical scavenger and also act to suppress the generation of active oxygen.

Specifically, the antioxidant is at least one selected from the group consisting of red wine yeast extracts, white wine yeast extracts, magnesium L-ascorbyl phosphate, hypotaurine, tocopherol, vanillin, catechin extracts, dibutylhydroxytoluene, coffee extracts, and dl-α-tocopherol acetate. Neither red wine extracts nor white wine extracts contain β-glucans.

The use of the antioxidant is thought to suppress oxidation of skin and prevent skin from aging.

The amount of the antioxidant to be blended is from 0.001 to 5%, preferably from 0.005 to 2% with respect to the total amount of all of the components of the skin care product. Desired effects are not obtained if the amount falls outside this range.

The term "deep sea water" as used herein refers to low-temperature, clean, and nutrient salt-rich water collected from the water layer that the surface sea water forms when carried to a certain depth. The deep sea water for use in the present invention is collected from a sea area that lies for example 1000 to 4000m and preferably 2000 to 3000m off the coast of the cape of Muroto, Kochi prefecture, Japan. The sea water is collected from a depth of 250 to 500m and preferably from a depth of 300 to 400m. The deep sea water as used herein also includes deep sea water processed through a membrane such as reverse-osmosis membrane.

Containing numerous kinds of minerals and having excellent permeability, the deep sea water is thought to have an excellent moisturizing ability and thus facilitate penetration of active ingredients blended in the skin care product through skin.

The amount of the deep sea water to be blended is from 50 to 99% and preferably from 50 to 90% with respect to the total amount of all of the components contained in the skin care product.

The skin care product of the present invention is characteristic in that it contains the N-acetylated basic amino acid compound and the antioxidant as essential ingredients or it contains as essential ingredients at least one selected from the N-acetylated basic amino acid compound, the antioxidant, and the deep sea water and yeast and/or yeast extracts. These components together exert synergistic effects to improve skin immunity.

The skin care product of the present invention may be used as cosmetic products, body cosmetic products, non-medical products, or the like and may be provided in the forms of milky lotion, cream, face lotion, facial pack, facial cleansing formula, massaging formula and in any other suitable form.

Aside from the above-described essential ingredients, the skin care product of the present invention may further contain other components that are commonly used in ordinary cosmetic products, provided that they do not affect the intended effects of the present invention. These components are properly selected depending on the purpose of the product. For example, the skin care product may contain lubricants, powder, surfactants, purified water, lower alcohols, polymers, gelling agents, UV-absorbing agents, UV-scattering agents, oxidation inhibitors, dyes, preservatives, flavors, cosmetic ingredients and any other suitable component.

### BEST MODE FOR CARRYING OUT THE INVENTION

The present invention will now be described in detail with reference to Examples, which are not intended to limit the scope of the invention in any way.

### Example 1 and Comparative Examples 1 and 2

To a basic formulation for the lotion shown in Table 1, materials shown in Table 2, namely, yeast extracts alone, N-acetylated basic amino acid alone, and a combination of yeast extracts and N-acetyl basic amino acid, were individually added to form three different lotions. The yeast extracts were obtained from brewer's yeast. The lotions so prepared were each applied to skin and the skin was measured for the amount of moisture it contained. The results are shown in Table 2.

**Table 1**

| Material blended | Percentage of the blend (wt %) |
|---|---|
| Lecithin | 1.0 |
| Cholesterol | 0.5 |
| Oleic acid | 0.5 |
| Glycerol | 3.0 |
| 1,3-butylene glycol | 7.0 |
| L-arginine | 0.25 |
| Glyceryl monooleate | 1.0 |
| Decaglyceryl monostearate | 1.5 |
| Oleyl alcohol | 0.5 |
| Propylene glycol didecanoate | 0.5 |
| Betaine | 0.5 |
| Isopropyl paramethoxy cinnamate | 2.0 |
| 2-(2' -hydroxy-5' -methylphenyl)benzotriazol | 2.0 |
| Purified water | Balance |

**Table 2**

| | Materials blended (wt %) | | Skin moisture (wt %) |
|---|---|---|---|
| | Yeast extracts | N-acetylarginine | |
| Example 1 | 0.03 | 0.5 | 28 |
| Comparative Example 1 | 0.03 | - | 20 |
| Comparative Example 2 | - | 0.5 | 25 |

As can be seen from the results shown in Table 2, the skin moisture obtained with the use of the lotion containing both yeast extracts and N-acetylarginine was significantly greater than that obtained with the use of the lotion containing the yeast extracts alone or N-acetylarginine alone.

### Example 2, and Comparative Examples 3 and 4

To the basic formulation for a lotion shown in Table 1, materials shown in Table 3, namely, yeast extracts alone, red wine yeast extracts alone, and a combination of yeast extracts and red wine yeast extracts, were individually added to form three different lotions.

The lotions so prepared were each applied to skin and the skin was then measured for the anti-oxidation activity (*i.e.*, % suppression of hydroxyl radicals) using DPPH (1,1-diphenyl-2-picrylhydrazyl).

**Table 3**

| | Materials blended (wt %) | | % suppression of hydroxyl radicals |
|---|---|---|---|
| | Yeast extracts | Red wine yeast extracts | |
| Example 2 | 0.03 | 0.1 | 51 |
| Comparative Example 3 | 0.03 | - | 15 |
| Comparative Example 4 | - | 0.1 | 42 |

As can be seen from the results shown in Table 3, the lotion containing both yeast extracts and red wine yeast extracts exhibited a higher suppression of hydroxyl radicals as compared to the lotion containing yeast extracts alone or the lotion containing red wine extracts alone.

### Example 3 and Comparative Example 5

To the basic formulation for the lotion shown in Table 1, materials shown in Table 4, namely, yeast extracts alone, deep sea water alone, and a combination of yeast extracts and deep sea water, were individually added to form three different lotions. The deep sea water was obtained by processing raw deep sea water through a reverse-osmosis membrane.

The lotions so obtained were each applied to faces of subjects and the subjects were visually evaluated for improvements in the degree of wrinkles.

For each lotion, 20 subjects with the lotion cream applied to their faces were evaluated. The subjects were evaluated independently by two evaluators for the degree of wrinkles in the adjacent areas of their mouths and eyes according to the packman SFL rating system. The results are shown in Table 4.

**Table 4**

| | Materials blended (wt %) | | Visually observed improvement in the degree of Wrinkles (%) |
|---|---|---|---|
| | Yeast extracts | Deep sea water | |
| Example 3 | 0.03 | 50.0 | 68 |
| Comparative Example 5 | 0.03 | - | 60 |

As can be seen from the results shown in Table 4, the lotion containing both yeast extracts and deep sea water exhibited a higher improvement in the degree of wrinkles as compared to the lotion containing yeast extracts alone.

### Example 4, and Comparative Examples 6 and 7

To the basic formulation for the lotion shown in Table 1, materials shown in Table 5, namely, N-acetylarginine alone, red wine yeast extracts alone, and a combination of N-acetylarginine and red wine yeast extracts, were individually added to form three different lotions.

The lotions so prepared were evaluated for improvements in the degree of wrinkles in the same manner as in Example 3. The results are shown in Table 5.

**Table 5**

| | Materials blended (wt %) | | Visually observed improvement in the degree of Wrinkles (%) |
|---|---|---|---|
| | N-acetylarginine | Red wine yeast extracts | |
| Example 4 | 0.5 | 0.1 | 72 |
| Comparative Example 6 | 0.5 | - | 55 |
| Comparative Example 7 | - | 0.1 | 63 |

As can be seen from the results shown in Table 5, the lotion containing both N-acetylarginine and red wine yeast extracts exhibited a higher improvement in the degree of wrinkles as compared to the lotion containing N-acetylarginine alone and the lotion containing red wine yeast extracts alone.

### Example 5

To the basic formulation shown in Table 1, 0.03% yeast extracts, 0.1% red wine yeast extracts, and 50.0% deep sea water processed by a reverse-osmosis membrane were added to form a lotion.

This lotion proved to exhibit excellent sunscreen effects, anti-dry skin effects, and anti-keratinizing effects.

### Example 6

A cream was prepared according to a formulation shown in Table 6 and through an ordinary method.

2g of the prepared cream was applied to the upper arm of a subject. The skin was collected and was exposed *in vitro* to hydroxyl radicals produced by breaking down H₂O₂ using Fe²⁺ as a catalyst. The results are shown in Table 7.

**Table 6**

| Materials blended | Percentage of the blend (wt %) |
|---|---|
| Yeast extracts | 0.015-0.045 |
| Red wine yeast extracts | 0.1 |
| N-acetylarginine | 0.5 |
| Deep sea water (processed through reverse osmosis membrane) | 50.0 |
| Jojoba oil | 10.0 |
| Soybean lecithin | 0.5 |
| Deoaglyceryl monostearate | 2.0 |
| Oleyl alcohol | 3.0 |
| Propylene glycol didecanoate | 0.5 |
| Carboxy vinyl polymer | 0.05 |
| Glycerol | 7.0 |
| 1,3-butylene glycol | 7.0 |
| L-arginine | 0.1 |
| Betaine | 0.5 |
| Preservative | 0.5 |
| Purified water | Balance |

**Table 7**

| | Concentration of yeast extracts (wt %) | | |
|---|---|---|---|
| | 0.015 | 0.030 | 0.045 |
| % suppression of hydroxyl radicals | 40 | 60 | 80 |

As can be seen from the results shown in Table 7, the cream containing yeast extracts, red wine yeast extracts, N-acetylarginine and deep sea water exhibited excellent free radical scavenging effects. It can also be seen that the free radical scavenging effects are enhanced as the concentration of yeast extracts is increased.

### Example 7

The cream prepared in Example 6 was applied to faces of subjects, and improvements in the degree of wrinkles were evaluated through visual inspection and image analysis after continual use of the cream for 0 to 6 weeks.

The visual evaluation was performed in the same manner as in Example 3.

The evaluation through image analysis was performed as follows. The cream was applied to the skin of 30 subjects and silicone replicas of the skin surfaces were prepared. 10 replicas were selected at random and were evaluated for the improvements in the degree of wrinkles through image analysis.

The results of the visual evaluation and image analysis are shown in Table 8.

**Table 8**

| Time (week) | % improvement in the degree of wrinkles | |
|---|---|---|
| | Visual evaluation | Evaluation through image analysis |
| 0 | 0 | 0 |
| 2 | 35 | 15 |
| 4 | 55 | 45 |
| 6 | 75 | 65 |

As can be seen from the results shown in Table 8, the cream containing yeast extracts, red wine yeast extracts, N-acetylarginine, and deep sea water exhibited an significant improvement in the degree of wrinkles.

### INDUSTRIAL APPLICABILITY

The skin care product of the present invention enhances skin immunity. In particular, the skin care product exhibits excellent anti-skin aging effects, including anti-dry skin effects, anti-kelatinizing effects, effects to promote skin turnover, and anti-wrinkle effects.

## Claims

1. A skin care product containing as essential ingredients an N-acetylated basic amino acid compound and an antioxidant.

2. A skin care product containing as essential ingredients at least one selected from an N-acetylated basic amino acid compound, an antioxidant, and deep sea water, and yeast and/or yeast extracts.

3. The skin care product according to claim 1 or 2, wherein the N-acetylated basic amino acid compound includes at least one selected from the group consisting of N-acetylarginine, N-α-acetyllysine, N-ε-acetyllysine, N-α-acetylornithine, N-γ-acetylornithine, N-α-acetylhistidine, and salts thereof.

4. The skin care product according to claim 1 or 2, wherein the antioxidant includes at least one selected from the group consisting of red wine yeast extracts, white wine yeast extracts, magnesium L-ascorbyl phosphate, hypotaurine, tocopherol, vanillin, catechin extracts, dibutylhydroxytoluene, coffee extracts, and dl-α-tocopherol acetate.
